(19) Europäisches Patentamt
European Patent Office
Office européen des brevets

(11) **EP 4 014 953 A1**

(12) **EUROPÄISCHE PATENTANMELDUNG**

(43) Veröffentlichungstag:
**22.06.2022 Patentblatt 2022/25**

(21) Anmeldenummer: **20215930.7**

(22) Anmeldetag: **21.12.2020**

(51) Internationale Patentklassifikation (IPC):
*A61K 8/14* (2006.01)   *A61K 8/9728* (2017.01)
*A61K 36/00* (2006.01)   *A61P 17/02* (2006.01)
*A61P 17/18* (2006.01)   *A61Q 17/00* (2006.01)
*A61Q 19/08* (2006.01)   *A61K 36/06* (2006.01)
*A61K 36/064* (2006.01)   *C12N 1/16* (2006.01)

(52) Gemeinsame Patentklassifikation (CPC):
**A61K 8/9728; A61K 8/14; A61K 36/064;**
**A61P 17/02; A61P 17/18; A61Q 17/00;**
**A61Q 19/08; C12N 1/16;** A61K 2800/412

(84) Benannte Vertragsstaaten:
**AL AT BE BG CH CY CZ DE DK EE ES FI FR GB**
**GR HR HU IE IS IT LI LT LU LV MC MK MT NL NO**
**PL PT RO RS SE SI SK SM TR**
Benannte Erstreckungsstaaten:
**BA ME**
Benannte Validierungsstaaten:
**KH MA MD TN**

(71) Anmelder: **Wohldorff GmbH**
**20354 Hamburg (DE)**

(72) Erfinder:
• **LINDEMANN, Björn**
**20459 Hamburg (DE)**
• **TOUREL, Sylvain**
**45525 Hattingen (DE)**

(74) Vertreter: **Simandi, Claus**
**Simandi Patentanwälte**
**Kurfürstendamm 45**
**10719 Berlin (DE)**

(54) **ZUSAMMENSETZUNGEN ENTHALTEND KOMAGATAELLA UND DEREN VERWENDUNG**

(57) Die Erfindung betrifft eine kosmetische oder dermatologische Zusammensetzung zur topischen Verwendung enthaltend Extrakte oder Kulturen aus Komagataella. Ebenfalls betrifft die Erfindung pharmazeutische oder dermatologische Zusammensetzung zur topischen Verwendung zur Behandlung von Hautatrophie, Verbrennungen, Wunden und Verletzungen.

zu Figur 1 - aus : Kurtzman et. al., Phylogenetic relationships among species of Pichia, Issachenkia and Williopsis determined from multigene sequence analysis, and the proposal of Barnettozyma gen.nov., Lindnera gen.nov. and Wickerhamomyces gen.nov. FEMS Yeast Res 8 (2008) 939-954.

Figur 2

**Beschreibung**

Die Erfindung betrifft eine kosmetische oder dermatologische Zusammensetzung zur topischen Verwendung enthaltend Extrakte oder Kulturen aus Komagataella. Ebenfalls betrifft die Erfindung eine pharmazeutische oder dermatologische Zusammensetzung zur topischen Verwendung enthaltend Extrakte oder Kulturen aus Komagataella zur Behandlung von Hauterkrankungen, Hautatrophie, Dermatitis, Verbrennungen, Wunden und Verletzungen.

Die Haut übt als das größte Organ des Menschen zahlreiche lebenswichtige Funktionen aus. Sie schützt z.B. vor Kälte, Hitze, Strahlung, dem Einwirken chemischer Substanzen und Krankheitserregern. Sofern die Haut diese Barrierefunktion nicht mehr ausreichend erfüllt, können lokale Irritationen oder auch ganzkörperliche Beschwerden erfolgen.

Die Haut, insbesondere die Epidermis, ist als Barriereorgan des menschlichen Organismus in besonderem Maße äußeren Einwirkungen unterworfen. Diese Barrierefunktion wird unter anderem von Hautlipiden aufrechterhalten. Diese epidermalen Lipide wie z.B. Glycosphingolipide, Ceramide, Sterine und Sterinester, Fettsäuren, Triglyceride, n-Alkane oder verschiedene polare Lipide werden im Keratinisierungsprozess freigesetzt.

Advanced Glycation Endproduct(s) (AGE) kommen ebenfalls in der Haut vor und bezeichnen mittels Glykation (Synonym: Glykierung) erhaltene Reaktionsprodukte, welche durch die Reaktion von Proteinen, Lipiden oder Nukleinsäuren mit Kohlenhydraten ohne Beteiligung von Enzymen erhalten werden, und zwar mittels Maillard Reaktion, Amadori Reaktion oder Schiffsche Base Reaktion (Ahmed N. Advanced glycation endproducts - role in pathology of diabetic complications. Diabetes Res Clin Pract 2005; 67:3-21).

Zu den am häufigsten in der Haut gebildeten AGEs gehören Carboxymethyllysin, Pentosidin, 3-Deoxyglucosom, Methylglyoxal, Glyoxal, Glucosepan, Carboxymethylhydroxylysin, Carboxyethyllysin, Fructoselysin, Methylglyoxal-abgeleitete Hydroimidazolone, Glyoxallysin, Methylglyoxallysin und Pyrralin. AGEs sind selbst reaktive Moleküle, die durch Interaktion mit ihren Rezeptoren verschiedene molekulare Signalwege in vivo aktivieren und auf diese Weise an Entzündung, Immunantwort, Zellproliferation und Genexpression beteiligt sind.

Die kutane Akkumulation von AGEs ist ein Merkmal der Hautalterung. Es werden in erster Linie langlebige Proteine in der Haut durch Glykation modifiziert. Die Kollagentypen I und IV, die eine langsame Umsatzrate von etwa 10 Jahren aufweisen, und andere dermale langlebige Proteine wie Fibronektin sind während der intrinsischen chronologischen Alterung in besonders hohem Maße von der Modifikation durch Glykation betroffen.

Die Glykation von Kollagen beeinträchtigt die Funktion auf verschiedene Weise. Intermolekulare Quervernetzungen benachbarter Kollagenfasern verändern die biomechanischen Eigenschaften, welche zu Steifigkeit und verminderter Biegsamkeit führen und folglich eine Anfälligkeit für mechanische Reize erhöht. Eine einhergehende Veränderung der Ladung und die Bildung von AGEs an Seitenketten des Kollagens beeinflussen die Kontaktstellen mit Zellen und anderen Matrixproteinen und hemmen die Fähigkeit mit ihnen zu reagieren. Durch Glykation modifiziertes Kollagen widersteht dem Abbau durch Matrix-Metalloproteinasen und hemmt in der Weise die Entfernung und den Ersatz durch neu synthetisiertes und funktional geeignetes Kollagen.

In fast allen untersuchten Organismen sind enzymatische Mechanismen zur Abwehr von AGEs gefunden worden. Das glutathionabhängige Glyoxalase-System - bestehend aus Glyoxalase I und II - übt eine Schlüsselrolle aus. Dieses System nutzt reduziertes Glutathion zur katalytischen Umwandlung von Glyoxal, Methylglyoxal und anderen $\alpha$-Oxoaldehyden in das weniger toxische D-Lactat. Andere enzymatische Systeme sind Fructosyl-Amin-Oxidasen (FAOXs) und Fructosamin-Kinasen, relativ neue Enzymklassen, die Amadori-Produkte - also AGEs - erkennen und spalten. FAOXs oder "Amadoriasen" werden jedoch nur in Bakterien, Hefen und Pilzen exprimiert, nicht jedoch in Säugetieren und Menschen.

In einem optimalen Zustand der Haut liegt ein ausgewogenes Verhältnis von Hautlipiden und Hautfeuchtigkeit vor. Durch dieses Gleichgewicht werden wichtige Eigenschaften der Haut, wie das Penetrationsvermögen, Wasserbindungsvermögen, Elastizität, Regenerationsfähigkeit oder die Widerstandsfähigkeit gegen Umwelteinflüsse und Noxen unterschiedlichster Art, bestimmt. Den Hautlipiden, besonders den Oberflächenlipiden, ist dabei eine übergeordnete Bedeutung zuzuschreiben. Die Alterung durch endogene Prozesse oder exogene Einflüsse oder anhaltende chemische oder physikalische Schädigung der Haut führt zu Störungen bei der Angiogenese, Lipid- und Schweiß-Produktion, Immunfunktion und Vitamin D Synthese und zur Anreicherung toxischer AGEs und verursacht Hauterkrankungen, insbesondere Hautatrophie und eine Verschlechterung der Wundheilung (Zouboulis CC, Makrantonaki E. Clinical aspects and molecular diagnostics of skin aging. Clin Dermatol 2011; 29:3-14).

Heutzutage ist die Haut vermehrt freien Radikalen - insbesondere Sauerstoff- und Stickstoffradikalen - ausgesetzt, wobei Luftverschmutzung (Stoßparameter) neben Sonnenstrahlung und Ozonbildung sowie chemischen Stoffen aus beispielsweise Reinigungs- und Desinfektionsmitteln und dekorativer Kosmetik die Menge und Reaktivität der freien Radikale erhöht. Als Resultat verursacht so genannter oxidativer Stress Hautschäden von der äußeren Schutzbarriere bis zu den unteren Hautschichten (Stratum corneum, Epidermis, Dermis und Hypodermis). Oxidativer Stress beschleunigt die Hautalterung und begünstigt Hauterkrankungen. Zudem wird die Wundheilung beeinträchtigt. Der Körper begegnet oxidativem Stress mit Antioxidantien als Radikalfängern.

Als wichtigste niedermolekulare Antioxidantien in der Haut gelten Vitamin C und E, die beide nicht vom Menschen synthetisiert werden können, sowie Glutathion, Thioredoxin und Liponsäure, die der menschliche Körper selbst bilden kann.

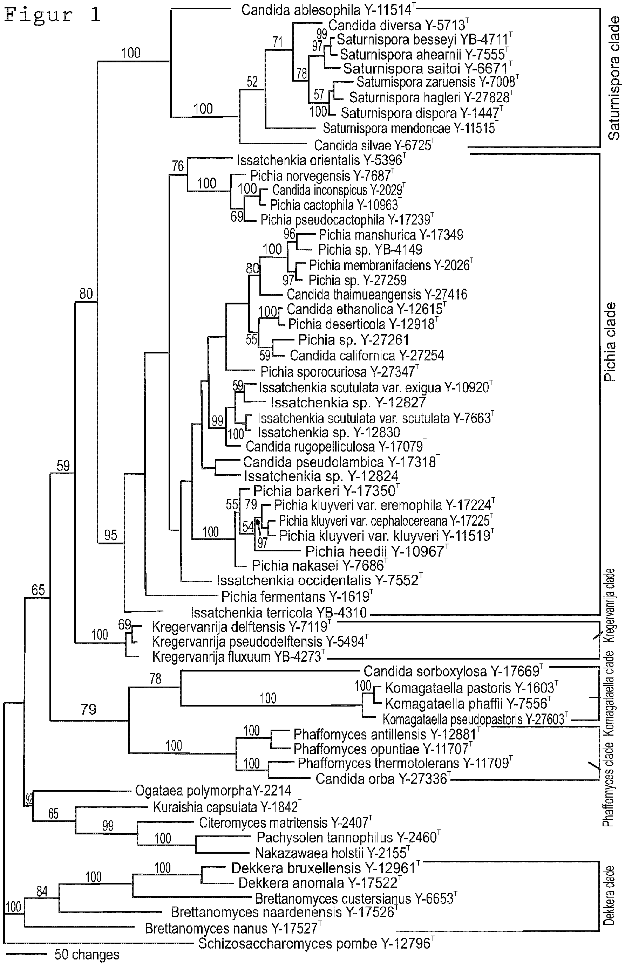

Die Hefe-Gattung Komagataella, insbesondere K. phaffii, gehört zu den sogenannten methylotrophen Hefen, welche Methanol als ausschließliche und einzige Energie- und Kohlenstoffquelle verwerten können. Fälschlicherweise wurde früher Komagataella mit Pichia gleichgesetzt und als Pichia klassifiziert. Kurtzman et al. (FEMS Yeast Res 8 (2008) 939-954, DOI:10.1111/j.1567-1364.2008.00419.x) und die dort gezeigte phylogenetische Stammbaumgrafik verdeutlicht (Figur 1), dass Pichia und Komagataella verschiedene Spezies sind.

Die Aufgabe der vorliegenden Erfindung besteht darin, eine dermatologische Zusammensetzung zur topischen Verwendung bereitzustellen.

Die Aufgabe wird durch mindestens einen Patentanspruch gelöst.

Die Erfinder konnten überraschender Weise feststellen, dass Extrakte oder Kulturen aus Komagataella oder Komagataella als solches, ggfs. lysiert, homogenisiert, zerkleinert oder gepresst, hervorragend in einer dermatologischen Zusammensetzung zur topischen Verwendung geeignet sind, und zwar zum einen für eine Kosmetik zum anderen für ein Pharmazeutikum, insbesondere für die Behandlung von Hauterkrankungen, insbesondere Hautatrophie, Dermatitis, Verbrennungen, Wunden und Verletzungen und zur Wundheilung.

Eine Dermatitis kann insbesondere aus der Gruppe periorale Dermatitis, Dermatitis ulcerosa, atopische Dermatitis, seborrhoische Dermatitis, Neurodermitis, Lichtdermatosen, Xerodermie, insbesondere Sebostase ausgewählt sein.

Die erfindungsgemäßen dermatologischen Zusammensetzungen zur topischen Verwendung enthaltend oder bestehend aus Extrakten oder Kulturen aus Komagataella zeigen hervorragende Eigenschaften sowohl als Antioxidantien als auch Mittel zur Reduktion von AGEs, sodass insbesondere der oxidative Stress und der AGE-Spiegel in der Haut vermindert werden kann und folglich sowohl als Kosmetikum oder als Pharmazeutikum, insbesondere zur Behandlung von Hauterkrankungen (Hautkrankheiten) geeignet sind.

Daher ist die vorgenannte erfindungsgemäße Zusammensetzung besonders geeignet, die Hautalterung zu verzögern, iSe. Anti-Ageing Mittels, siehe hierzu die Studie zur Faltenreduktion und die Advanced Glycation Endproduct (AGE) Versuche in den Beispielen.

Daher betrifft die Erfindung in einer bevorzugten Ausführungsform eine kosmetische oder dermatologische Zusammensetzung zur topischen Verwendung enthaltend oder bestehend aus Extrakten oder Kulturen aus Komagataella, wobei die Zusammensetzung ein Anti-Ageing Mittel ist oder ein Mittel zur Reduzierung von oxidativem Stress und oder ein Mittel zur Reduktion von Advanced Glycation Endproduct(s) (AGEs).

Weiterhin ist die vorgenannte erfindungsgemäße Zusammensetzung besonders geeignet, die Entstehung von Falten zu verhindern und bereits vorhandene Falten zu reduzieren, insbesondere die Faltenbildung zu verhindern, siehe Beispiele.

Daher betrifft die Erfindung in einer bevorzugten Ausführungsform eine kosmetische oder dermatologische Zusammensetzung zur topischen Verwendung enthaltend oder bestehend aus Extrakten oder Kulturen aus Komagataella, wobei die Zusammensetzung ein Mittel zur Prophylaxe oder Verhinderung der Entstehung von Falten und zur Reduktion von bereits vorhandenen Falten, insbesondere zur Verhinderung der Faltenbildung ist.

Weiterhin betrifft die Erfindung in einer bevorzugten Ausführungsform ein Mittel zur Reduktion von Mund-, Nasen-, Stirn-, Kinn-, Augen-, Hals- und Dekollete -Falten und Augenringen.

In einer weiteren bevorzugten Ausführungsform der Erfindung sind solche Komagataella umfasst, welche ausschließlich Methanol als einzige Energie- und Kohlenstoffquelle erhalten haben. Während der Kultivierung der Komagataella wurde die Konzentration des für alle Organismen metabolisch toxischen Methanols stufenweise erhöht, so dass die Komagataella-Zellen einem wachsenden toxischen und oxidativen Stress ausgesetzt wurden. Am Anfang dieser Kultivierung entnommene Komagataella-Zellen sind in der Endkonzentration von Methanol im Gegensatz zu den durch gesteigerte Konzentrationen angepasste Zellen nicht überlebensfähig. Diese stressinduzierten und - optimierten Komagataella und deren Extrakte und Kulturen erweisen sich erfindungsgemäß besonders geeignet, sowohl den oxidativen Stress und den AGE-Spiegel der Haut als auch die Faltenbildung sowie bereits vorhandene Falten zu reduzieren.

Im Sinne dieser Erfindung umfasst der Begriff Komagataella, insbesondere solche methylotrophe Komagataella spp., insbesondere die Arten K. phaffii, K. pastoris, K. pseudopastoris, bevorzugt ist jedoch K. phaffii.

Die erfindungsgemäßen Kulturen oder Extrakte können aus wässrigen oder üblichen Kulturen von Komagataella gewonnen werden, ggfs. solche, welche Methanol als einzige Energie- und Kohlenstoffquelle erhalten haben, und nach Anreicherung können die Kulturen oder Extrakte bzw. Biomasse entnommen und in eine Trockenmasse überführt werden. Weiterhin kann eine Gefriertrocknung vorgesehen sein und die erhaltene Trockenmasse in ein Pulver überführt werden. In einer weiteren bevorzugten Ausführungsform wird die Trockenmasse zerkleinert, lysiert, homogenisiert oder gepresst, oder die erhaltenen Kulturen oder Extrakte bzw. Biomasse zerkleinert, homogenisiert, gepresst oder lysiert (z.B. French-Presse) und anschließend getrocknet. Im Ergebnis werden die Zellen von Komagataella ganz oder teilweise aufgeschlossen (sog. Zellaufschluss, wobei Zellmembranen zerstört werden).

Die Kultivierung erfolgt aerob (in Gegenwart von Sauerstoff) z.B. in einem Bioreaktor oder Fermenter. Beispielsweise wird mit Glucose als Kohlenstoffquelle fermentiert, bis die vorgelegte Menge Glucose verbraucht ist, welches am rapiden Ansteigen der gelösten, nicht verbrauchten Konzentration von Sauerstoff erkennbar ist. Dann wird Glucose im Zulaufverfahren in limitierender Rate zugefüttert, entweder für eine bestimmte Zeit oder bis eine bestimmte Zelldichte an Komagataella erreicht ist. Anschließend wird das Zulaufverfahren in einer bevorzugten Ausführungsform der Erfindung auf Methanol als einzige und ausschließliche Kohlenstoffquelle umgestellt. Um zu verhindern, dass Methanol zu toxischen Konzentrationen führt, wird die Methanolzufuhr in regelmäßigen Abständen gestoppt und die Zeit bestimmt, die bis zum Ansteigen der gelösten Sauerstoffkonzentration verstreicht. Sodann wird die Methanolzufuhr fortgeführt und gesteigert, so dass eine stufenweise höhere Methanolkonzentration im Bioreaktor erzielt wird.

Ein "Bioreaktor" im Sinne dieser Erfindung kann synonym als Fermenter bezeichnet werden und dient zur Kultivierung der Komagataella zur Herstellung von Biomasse bzw. angereicherten Kulturen oder Extrakten, wobei erfindungsgemäß ein kontinuierlicher Betrieb des Bioreaktors bevorzugt ist. Der Fachmann ist in der Lage, für Komagataella entsprechende Betriebsparameter beispielsweise über ein Mess- und Steuerungssystem einzustellen (Temperatur, pH-Wert der Kulturlösung, etc.) und Kulturmedien bereitzustellen.

Als vorteilhafte C-Quelle in einem Kulturmedium können Einfach- und/oder Mehrfachzucker, und zwar insbesondere Glucose, in einer Konzentration von 0,3 bis 10 g/L Kulturmedium zugegeben werden.

In einer weiteren Ausführungsform kann die erfindungsgemäße kosmetische oder dermatologische Zubereitung zur topischen Verwendung in Form eines Gels, Sprays, Shampoos, einer Salbe, Creme, Lotion (Hautmilch), Paste oder vorzugsweise Emulsion erfolgen, wobei vorzugsweise wässrige Systeme verwendet werden.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert. Sind die beiden Flüssigkeiten Wasser und Öl und liegen Öltröpfchen fein verteilt in Wasser vor, so handelt es sich um eine Öl-in-Wasser-Emulsion (O/W-Emulsion). Der Grundcharakter einer O/W-Emulsion ist durch das Wasser geprägt. Bei einer Wasser-in-Öl-Emulsion (W/O-Emulsion) handelt es sich um das umgekehrte Prinzip, in dem der Grundcharakter hier durch das Öl bestimmt wird. Weiterhin sind Mischsysteme wie Wasser-in-Öl-in-Wasser-Emulsionen (W/O/W-Emulsion) und Öl-in-Wasser-in-Öl-Emulsionen (O/W/O-Emulsionen) bekannt. Alle genannten Emulsionen sind erfindungsgemäß geeignet.

Emulgatoren sind Hilfsstoffe, die dazu dienen, die beiden nicht oder nur begrenzt miteinander mischbaren Flüssigkeiten zur Emulsion zu vermengen und zu stabilisieren.

Emulgatoren können vorteilhaft gewählt werden aus der Gruppe der nichtionischen, anionischen, kationischen oder amphoteren Emulgatoren.

Als nichtionischer Emulgator können verschiedene Emulgatoren aus den Gruppen der Partialfettsäureester, Fettalkohole, Sterole, Polyethylenglycole wie z.B. ethoxylierte Fettsäuren, ethoxylierte Fettalkohole und ethoxylierte Sorbitanester, Zuckeremulgatoren, Polyglycerinemulgatoren oder Silikonemulgatoren Verwendung finden.

Als anionischer Emulgator können verschiedene Emulgatoren aus den Gruppen der Seifen z. B. Natriumstearat, Fettalkoholsulfate, Mono-, Di- und Trialkylphosphosäureester und deren Ethoxylate, Fettsäure Lactat Ester, Fettsäure Citrat Ester oder Fettsäure Citroglycerinester Verwendung finden.

Als kationischer Emulgatoren können beispielsweise quaternäre Ammoniumverbindungen mit einem langkettigen aliphatischen Rest z. B. Distearyldimoniumchlorid eingesetzt werden.

Unter den amphoteren Emulgatoren können verschiedene Emulgatoren aus den Gruppen Alkylamininoalkancarbonsäuren, Betaine, Sulfobetaine oder Imidazolinderivate Verwendung finden.

Erfindungsgemäß bevorzugt sind natürlich vorkommende Emulgatoren, zu denen beispielsweise u.a. Bienenwachs, Wollwachs, Lecithin und Sterole gehören, die ebenfalls bei der Herstellung einer erfindungsgemäßen Zubereitung eingesetzt werden können. In einer bevorzugten Formulierung der erfindungsgemäßen Zubereitung können O/W-Emulgatoren aus der Gruppe der Pflanzenproteinhydrolysate und deren Derivate ausgewählt werden.

In einer weiteren bevorzugten Ausführungsform können die Kulturen oder Extrakte bzw. Biomasse aus Komagataella in Mikrokapseln, Mikropartikel oder Liposomen vorliegen. Dies erlaubt die erleichterte Herstellung von Emulsionen.

Im Sinne der vorliegenden Erfindung können ferner vorteilhaft Substanzen als Zusatzstoffe enthalten sein, ausgewählt aus der Gruppe der Ester aus gesättigten und/oder ungesättigten, verzweigten und/oder unverzweigten Alkancarbonsäuren und/oder Alkencarbonsäuren mit einer Kettenlange von 3 - 30 Kohlenstoffatomen und gesättigten und/oder ungesättigten, verzweigten und/oder unverzweigten Alkoholen einer Kettenlange von 3 - 30 Kohlenstoffatomen sowie aus der Gruppe der Ester aus aromatischen Carbonsäuren und gesättigten und/oder ungesättigten, verzweigten und/oder unverzweigten Alkoholen einer Kettenlange von 3 bis 30 C-Atomen. Solche Esterole können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononyliso-nonanoat, 2-Ethylhexylpalmitat, 2-Ethylhexyllaurat, 2- Hexyldecylstearat, 2-Octyldodecyl-palmitat, Oleyloleat, Oleylerucat, Erucyloleat, Erucylerucat sowie synthetische, halbsynthetische und natürliche Gemische solcher Ester, wie z.B. Jojobaöl.

Die Erfindung betrifft ebenfalls eine erfindungsgemäße Zubereitung, die als Zusatzstoffe, beispielsweise Jojobaöl, Olivenöl, Sonnenblumenöl, Sojaöl, Erdnussöl, Rapsöl, Mandelöl, Palmöl, Kokosöl, Palmkernöl oder dergleichen enthält. Ganz besonders bevorzugt sind pflanzliche Öle, die mindestens 6 Gew.-% an mehrfach ungesättigten Fettsäuren aufweisen.

Insbesondere können in den erfindungsgemäßen Zusammensetzungen zusätzlich als Hilfs- oder Zusatzstoff Antioxidantien und/oder Radikalfänger zugesetzt werden. Vorteilhaft werden solche Antioxidantien ausgewählt aus der Gruppe der lipophilen Systeme, wie beispielsweise: natürliche und synthetische Tocopherole (Vitamin E), Retinol (Vitamin A), Nordihydroguajaretsäure, Coniferylbenzoat, Butylhydroxyanisol, Gallussäureester und verschiedene antioxidative Pflanzenextrakte. Unter den hydrophilen Systemen sind besonders vorteilhaft Cystein oder Ascorbinsäure (Vitamin C) zu verwenden. Ebenfalls können Glutathion, Thioredoxin oder Liponsäure vorgesehen sein.

Weiterhin können die Antioxidantien ausgewählt sein aus Carotenoid-Terpenoiden, wie Astaxanthin, Canthaxanthin, Lutein, Lycopen, Zeaxanthin, oder Polyphenole, wie Apigenin, Luteolin, Tangeritin, Isorhamnetin, Kaempferol, Myricetin, Proanthocyanidine, Tannine, Quercetin, Eriodictyol, Hesperetin, Naringennin, Catechin, Gallocatechin, Epicatechin, Epigallocatechin, Isoflavone, Genistein, Glycitein, Resveratrol, Anthocyanidine, inkl. Delphinidin, Malvidin als auch Phenolsäuren und Ester davon, wie Rosmarinsäure u.a. oder Pflanzenextrakte.

Den erfindungsgemäßen Zusammensetzungen können zusätzlich lebende Mikroorganismen, die Teil des natürlichen und ausgewogenen Hautmikrobioms sein können, oder auch Extrakte von Mikroalgen wie Euglena gracilis, Chlorella vulgaris oder Spirolina maxima zugesetzt werden. Mikroalgen sind reich an Nährstoffen (Aminosäuren und Kohlenhydrate) und enthalten enzymatische und photoaktive Substanzen. Beispielsweise enthalten Spirolina-Extrakte Photolyasen, die UV-induzierte Nucleosid-Dimere der DNA in den Hautzellen reparieren und Phycocyanine, die schädliches, energiereiches blaues Licht reflektieren können.

Die erfindungsgemäßen kosmetischen Zusammensetzungen können weiterhin kosmetische Hilfsstoffe enthalten, wie sie üblicherweise in solchen Zubereitungen verwendet werden, z. B. Coenzym Q10, Konservierungsmittel, Bakterizide, Fungizide, Viruzide, Parfüme, Farbstoffe, Pigmente, die eine färbende Wirkung haben, Verdickungsmittel, oberflächenaktive Substanzen, weichmachende, anfeuchtende und/oder feuchthaltende Substanzen oder andere übliche Bestandteile einer kosmetischen Formulierung wie Alkohole, Polyole, Polymere, Schaumstabilisatoren, Elektrolyte, organische Lösemittel.

Auf der Basis der vorgenannten Formulierung kann ebenfalls ein erfindungsgemäßes pharmazeutisches Mittel zur topischen Verwendung bereitgestellt werden.

Die Erfindung soll nun im Folgenden anhand von Beispielen verdeutlicht werden, ohne jedoch auf diese Beispiele beschränkt zu werden.

Beispiel 1:

Tiefgekühlte Komagataella (-80° C) wurden in 100 ml YNMB vorkultiviert und bei 28° und 230 rpm für maximal 24 h geschüttelt und in einen Bioreaktor überführt. Das Einimpfungsvolumen betrug ungefähr 10 % des finalen Startvolumens.

Batch-Kultivierung

Die Batch-Kultivierung wurde in einem 5 l Glasbioreaktor durchgeführt. Das Basalsalzmedium wurde sterilisiert und der pH-Wert des Bioreaktors wurde nach Autoklavierung auf pH 5,0 mit Hilfe von Salzsäure und Ammoniumhydroxid eingestellt und konstant gehalten. Die gelöste Sauerstoffkonzentration wurde mit einer Elektrode fortwährend gemessen. Die Kultivierung erfolgte bei 28° unter Rühren bei 1200 rpm.

Zur Herstellung von Batch-Kulturen wurde nach Anfüttern mit Glucose und Nachweis der Erhöhung der gelösten Sauerstoffkonzentration stoßweise eine 1 %-ige Methanollösung (v/v) hinzugegeben, wie bereits oben beschrieben.

Das Trockengewicht wurde nach Zentrifugation von entnommener 5 ml Kulturbrühe (5000 rpm bei 4 °C für 10 Minuten) mit 5 ml Aqua dest. und Trocknen bei 105° in einem Ofen bestimmt.

Beispiel 2:

Tabelle 1. Beispielhafte Rezeptur für eine erfindungsgemäße topische Verwendung:

| Inhaltsstoffe | Menge in g oder Volumen in ml |
|---|---|
| Lecithin | 20 g |

(fortgesetzt)

| Inhaltsstoffe | Menge in g oder Volumen in ml |
|---|---|
| Ethanol | 40 ml |
| NaCl | 2 g |
| PEG 8k | 2 g |
| Glycerin | 5 g |
| Komagataella Extrakt | 10 ml |
| Mandelöl | 20 ml |
| Sesamöl | 10 ml |
| Sheabutter | 50g |
| Steriles Wasser | Auf 100 ml |

Zur Herstellung einer Emulsion kann die wässrige Phase durch Auflösen von PEG 8k (Polyethylenglycol)in Wasser und anschließend mit NaCl und Glycerin unter Rühren versetzt werden.

Zur Herstellung der organischen Phase wird Lecithin, Sesamöl, Mandelöl und Sheabutter in einem 250 ml Messbecher mit Ethanol versetzt und bei 70° im Wasserbad gerührt.

Die wässrige Phase wird langsam zur organischen Phase unter Rühren und Mixen (auch Ultraschall) hinzugegeben und die erhaltene Emulsion wird homogenisiert (z.B. durch Ultraschall). Die erhaltene Emulsion ist zur topischen Verwendung geeignet. Der Komagataella-Extrakt liegt in Liposomen vor.

Beispiel 3:

Das Advanced Glycation Endproduct (AGE) dient zum in vivo Nachweis von Alterung und oxidativem Stress der Haut, wobei mit einem spektroskopischen AGE-Reader (z.B. DiagnOptics B.V., Groningen (NL)) der Gehalt an AGEs anhand deren Autofluoreszenz bestimmt wird (Meerwaldt R, Links T, Graaff R, Thorpe SR, Baynes JW, Hartog J, et al. Simple noninvasive measurement of skin autofluorescence. Ann N Y Acad Sci 2005; 1043:290-8; Mulder DJ, Water TV, Lutgers HL, Graaff R, Gans RO, Zijlstra F, et al. Skin autofluorescence, a novel marker for glycemic and oxidative stress-derived advanced glycation endproducts: an overview of current clinical studies, evidence, and limitations. Diabetes Technol Ther 2006).

Ebenfalls kann der AGE Gehalt bzw. die Autofluoreszenz-Intensität zum Nachweis der Wirkung für ein Anti-Ageing Mittel herangezogen werden (Beisswenger PJ, Howell S, Mackenzie T, Corstjens H, Muizzuddin N, Matsui MS. Two fluorescent wavelengths, 440(ex)/520(em) nm and 370(ex)/440(em) nm, reflect advanced glycation and oxidation end products in human skin without diabetes. Diabetes Technol Ther 2012; 14:285-92).

In diesem Zusammenhang wurde ein Experiment mit einer Person durchgeführt, an der das Vorhandensein von hautassoziierten AGEs über einen Zeitraum von 33 Tagen mit einem vorgenannten AGE-Reader gemessen wurde. In der Testphase wurde täglich die Autofluoreszenz der Haut an beiden Unterarmen der Testperson bestimmt, einer gesunden Frau, 48 Jahre alt, Raucherin. Die Testperson behandelte ihren linken Unterarm einmal täglich mit der Hautcreme, hergestellt nach Beispiel 2, während sie ihren rechten Unterarm unbehandelt ließ. Die Messungen wurden zu identischen Zeitpunkten durchgeführt.

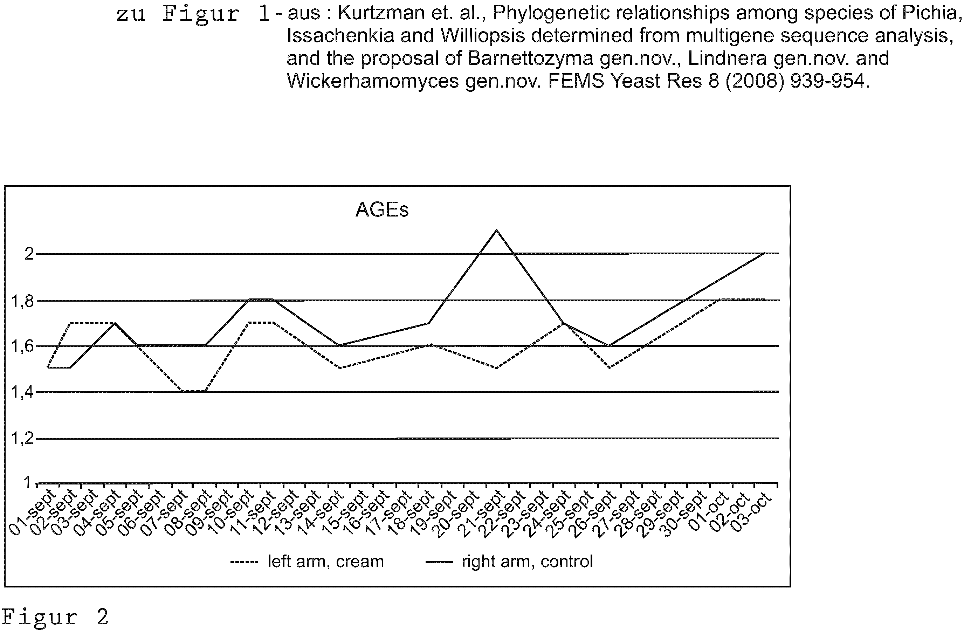

Die Ergebnisse sind in Figur 2 dargestellt.

Die Figur 2 zeigt die Entwicklung der Hautautofluoreszenzintensitäten an beiden Unterarmen während des Testzeitraums der Testperson mit der gestrichenen Kurve, die den mit Hautcreme behandelten linken Unterarm darstellt, und der durchgezogenen Kurve, die den unbehandelten rechten Kontrollunterarm zeigt.

Die Autofluoreszenzintensitäten variieren von Tag zu Tag aufgrund von Essgewohnheiten, täglicher körperlicher Bewegung, Außentemperatur, Wetterbedingungen usw. Daher zeigen die Kurven der Testperson unterschiedliche Intensitäten, diese verlaufen jedoch über den gesamten Testzeitraum parallel, wobei die Fluoreszenzintensitäten des linken Creme-behandelten Unterarms im Vergleich zum unbehandelten Kontrollunterarm niedriger sind, was einer verminderten Konzentration von AGEs in der behandelten Unterarmhaut entspricht. Ausgehend von einem identischen Autofluoreszenzniveau am Tag 0 (01. September) sinkt die Konzentration von Glykationsprodukten in der Haut, die die Cremebehandlung erhalten hat, am 20. Tag (21 September) schnell um bis zu 40 %.

Folglich ist die Eignung von Komagataella, insbesondere in Form einer erfindungsgemäßen Zusammensetzung sowohl als Mittel zur Reduzierung des oxidativen Stresses als auch Anti-Ageing Mittel gezeigt.

Beispiel 4:

Studie mit einer erfindungsgemäßen Rezeptur / Formulierung Ziel der Studie war es, die Wirksamkeit der Formulierungen (Prüfpräparate A und B) in Bezug auf die Reduzierung des Erscheinungsbildes von Gesichts-Falten und -Ausdruckslinien, die Verbesserung der Hautfeuchtigkeit und das Auftreten von Nebenwirkungen (Hautreizungen) nach kontinuierlicher Anwendung über einen Zeitraum von bis zu 56 Tagen zu beurteilen.

Prüfpräparat A ist eine Hautcreme entsprechend Beispiel 1, jedoch ohne Komagataella-Extrakt (Kontrolle), Prüfpräparat B ist die Hautcreme wie Prüfpräparat A, jedoch mit Komagataella-Extrakt (Verum).

Versuchspersonen: n = 11, mittleres Alter: 52 ± 4 Jahre, Phototyp (Fitzpatrick): 9% Phototyp II und 91% Phototyp III.

Die Versuchspersonen wurden angewiesen, 15 Tage vor dem ersten Tag der Studie und während der Studie keine Kosmetikprodukte mehr im Gesicht zu verwenden.

Das Prüfpräparat A wurde auf einer Seite des Gesichts und das Prüfpräparat B auf der anderen Seite aufgetragen. Die Produkte wurden zweimal täglich auf die saubere und trockene Gesichtshaut an derselben Stelle aufgetragen: einmal am Morgen und einmal am Abend. Die Produkte wurden mit Aufwärtsbewegungen verteilt, bis sie eingezogen waren.

Die sensorische Beurteilung durch die klinische Wirksamkeit erfolgte durch einen Dermatologen zu Beginn der Studie, nach 28 und 56 Tagen häuslicher Anwendung der Prüfpräparate.

Die Reduktion der Falten und Ausdruckslinien wurde durch die klinische Bewertung zur Klassifizierung der Falten nach ihrer Intensität durchgeführt. Diese Bewertung erfolgt anhand einer 9-Punkte-Skala, die auf Standardbildern basiert, die jeweils die Intensität der Falten repräsentieren, wie folgt: 1 = nicht vorhanden, 2 = schwach ausgeprägt, 3 = ausgeprägt, 4 = etwas, 5 = mäßig, 6 = nicht intensiv, 7 = intensiv, 8 = sehr intensiv, 9 = stark.

Durch diese Klassifizierung ist es möglich, die Verringerung der Intensität der Falten und Ausdruckslinien zu beurteilen, und das Prüfpräparat bietet zufriedenstellende Ergebnisse, wenn sich im Vergleich zu den anfänglichen Zuständen der Haut eine signifikante Verringerung der Falten nach der Behandlung zeigt. Der Prozentsatz der Faltenreduzierung wird anhand der zeitlichen Durchschnittswerte berechnet, die im Ausgangszustand (Baseline) und im Endzustand (nach der Behandlung) gemäß Gleichung 1 erhalten wurden:

$$\%RW = 100\left(\frac{IW_{ti} - IW_{t0}}{IW_{ti}}\right)$$

Berechnung der prozentualen Reduktion der Falten (%). IW = Ausgangswert der Faltenintensität (t0) oder nach ti (i = nach der Behandlung mit dem Produkt).

Tabelle 2 zeigt die Ergebnisse, die bei der klinischen Bewertung der Intensität der Falten und Ausdruckslinien zu Beginn, nach 28 und 56 der In-Home-Nutzung der Prüfprodukte A und B erzielt wurden.

| Tabelle 2. Ergebnisse der Klassifizierung der Intensitäten von Falten und Ausdruckslinien | | | | | | | | | | |
|---|---|---|---|---|---|---|---|---|---|---|
| | Prüfprodukt A | | | | | Prüfprodukt B | | | | |
| | | | | [%] Verbesserung* | | | | | [%] Verbesserung* | |
| Proband | Tag 0 | Tag 28 | Tag 56 | Tag 28 | Tag 56 | Tag 0 | Tag 28 | Tag 56 | Tag 28 | Tag 56 |
| 01 | 4.0 | 3.5 | 3.5 | 12.5 | 12.5 | 4.0 | 3.5 | 3.5 | 12.5 | 12.5 |
| 02 | 6.5 | 6.5 | 6.5 | 0.0 | 0.0 | 7.0 | 7.0 | 7.0 | 0.0 | 0.0 |
| 03 | 5.0 | 5.0 | 5.0 | 0.0 | 0.0 | 5.0 | 5.0 | 4.5 | 0.0 | 10.0 |
| 04 | 4.5 | 4.5 | 4.5 | 0.0 | 0.0 | 4.5 | 4.5 | 4.0 | 0.0 | 11.1 |
| 07 | 5.0 | 5.0 | 5.0 | 0.0 | 0.0 | 5.0 | 4.5 | 4.5 | 10.0 | 10.0 |
| 08 | 4.0 | 4.0 | 4.0 | 0.0 | 0.0 | 4.0 | 4.0 | 3.5 | 0.0 | 12.5 |
| 09 | 5.0 | 5.0 | 5.0 | 0.0 | 0.0 | 5.5 | 5.0 | 5.0 | 9.1 | 9.1 |
| 11 | 4.5 | 4.0 | 4.0 | 11.1 | 11.1 | 4.5 | 4.5 | 4.0 | 0.0 | 11.1 |
| 12 | 4.0 | 4.0 | 4.0 | 0.0 | 0.0 | 4.0 | 4.0 | 3.5 | 0.0 | 12.5 |
| 13 | 4.5 | 4.5 | 4.0 | 0.0 | 11.1 | 4.0 | 4.0 | 4.0 | 0.0 | 0.0 |
| 15 | 4.5 | 4.5 | 4.5 | 0.0 | 0.0 | 5.0 | 4.5 | 4.5 | 10.0 | 10.0 |
| Mittelw. | 4.7 | 4.6 | 4.5 | 2.1 | 3.2 | 4.8 | 4.6 | 4.4 | 3.8 | 9.0 |
| Std.abw. | 0.7 | 0.8 | 0.8 | 4.8 | 5.4 | 0.9 | 0.9 | 1.0 | 5.3 | 4.6 |
| Reaktive Proban-den [%] | – | – | – | 18 | 27 | – | – | – | 36 | 82 |

* Prozent Verbesserung = 100* (Startwert – Endwert) / Startwert, Startwert = Tag 0, Endwert = Tag 28 oder 56.

Die bei der klinischen Ausgangsbeurteilung ermittelten Werte wurden mit den Werten verglichen, die nach 28- und 56-tägiger Behandlung mit den Prüfpräparaten erzielt wurden, und zwar mit der Methode des t-Student-Tests, bimodal, gepaart, unter Berücksichtigung eines Konfidenzintervalls von 95%. Nach den Ergebnissen der statistischen Analyse gab es keine signifikante Reduktion der Intensität von Falten und Mimikfalten (P>0,05) nach 28 und 56 Tagen häuslicher Anwendung des Prüfpräparates A (Kontrolle).

Für das Prüfpräparat B (Verum) konnte eine signifikante Reduktion der Intensität von Falten und Ausdruckslinien (P<0,05) nach 28 und 56 Tagen häuslicher Anwendung beobachtet werden. Es konnte eine signifikante Abnahme der Intensität von Falten und Ausdruckslinien von 3,8% nach 28 Tagen und von 9,0% nach 56 Tagen häuslicher Anwendung des Prüfpräparates B beobachtet werden. Es konnte auch nachgewiesen werden, dass 36% und 82% der Probanden eine Abnahme der Intensität von Falten und Ausdruckslinien nach 28 bzw. 56 Tagen häuslicher Anwendung des Prüfpräparates B aufwiesen.

Beispiel 5:

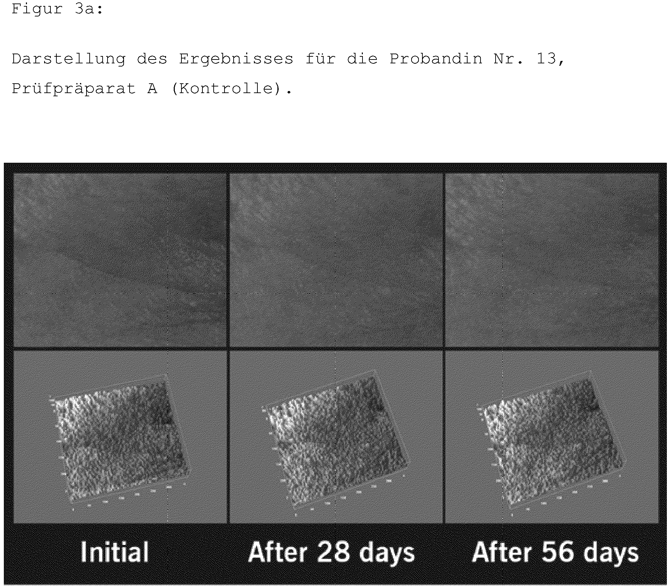

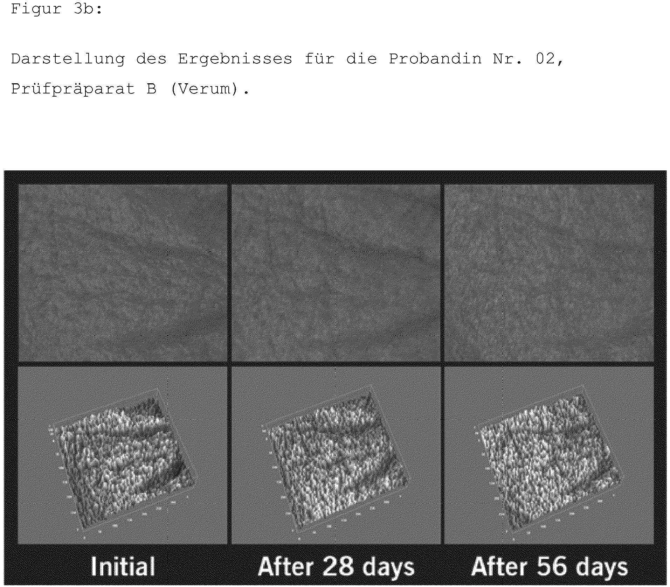

Auswertung der Behandlung mit den Prüfpräparaten A (Kontrolle) und B (Verum) aus Beispiel 4 mit einem digitalen bildgebenden Verfahren, siehe Figuren 3a und 3b.

Die Auswertungen basierten auf hochauflösenden digitalen Fotografien unter kontrollierten technischen Bedingungen - zu Beginn der Studie, nach 28 und 56 Tagen der In-Home-Anwendung der Prüfpräparate. Für die Positionierung der Probanden wurde eine Halterung verwendet, um das Gesicht für die Aufnahmen als Porträt oder Nahaufnahme reproduzierbar zu fixieren.

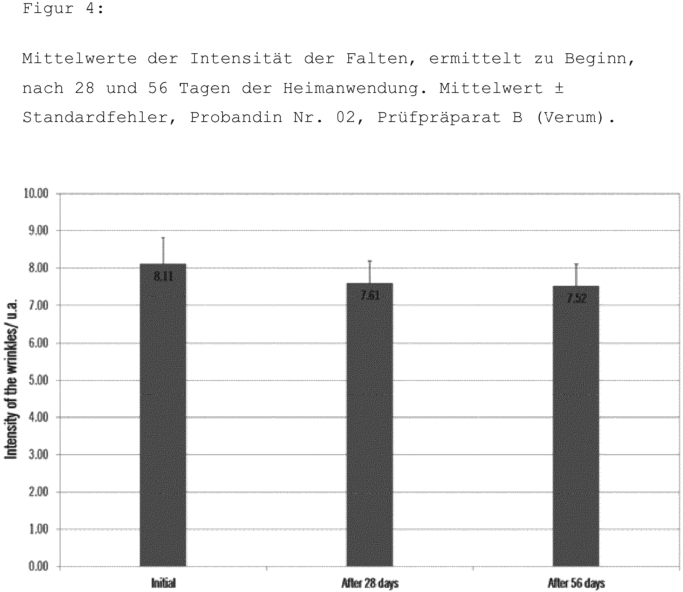

Die Ergebnisse sind in Figur 4 dargestellt.

Beispiel 6:

Behandlung von Augenfalten mit der Hautcreme aus Beispiel 2 mit Komagataella-Extrakt

Mehrere Probanden mit ausgeprägten Augenfalten wurden 6 Wochen lang mit der Hautcreme entsprechend Beispiel 2 mit Kamagataella-Extrakt behandelt. Die Creme wurde 2x täglich in üblicher Menge auf die Bereiche der Lachfalten im Augenbereich aufgetragen. Andere kosmetische Präparationen wurden nicht angewendet. Zu Behandlungsbeginn, nach zwei und nach 6 Wochen wurden Aufnahmen der Falten gemacht. Die Abbildungen zeigen zwei weibliche und einen männlichen Probanden.

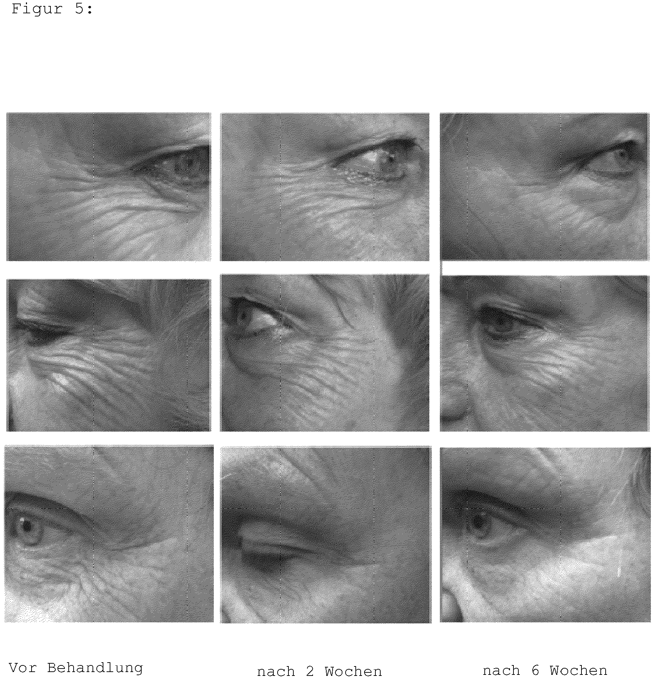

Siehe Figur 5.

**Patentansprüche**

1. Kosmetische oder dermatologische Zusammensetzung zur topischen Verwendung enthaltend Komagataella.

2. Kosmetische oder dermatologische Zusammensetzung zur topischen Verwendung enthaltend Komagataella, welches Methanol als einzige Energie- und Kohlenstoffquelle erhalten haben.

3. Kosmetische oder dermatologische Zusammensetzung zur topischen Verwendung nach Anspruch 2, wobei die Komagataella durch steigende Methanolkonzentrationen oxidativ gestresst wurde.

4. Kosmetische oder dermatologische Zusammensetzung zur topischen Verwendung nach einem der vorstehenden Ansprüche, wobei die Zusammensetzung ein Anti-Ageing Mittel ist oder ein Mittel zur Reduzierung von oxidativem Stress oder ein Mittel zur Reduktion von Advanced Glycation Endproduct(s) (AGEs).

5. Kosmetische oder dermatologische Zusammensetzung zur topischen Verwendung nach einem der vorstehenden Ansprüche, wobei die Zusammensetzung ein Mittel zur Prophylaxe oder Verhinderung der Entstehung von Falten und zur Reduktion von bereits vorhandenen Falten, insbesondere zur Verhinderung der Faltenbildung, insbesondere ein Mittel zur Reduzierung von Mund-, Nasen-, Stirn-, Kinn-, Augen-, Hals-, Dekollete-Falten und Augenringen ist.

6. Pharmazeutische oder dermatologische Zusammensetzung zur topischen Verwendung enthaltend Komagataella.

7. Pharmazeutische oder dermatologische Zusammensetzung zur topischen Verwendung enthaltend Komagataella, welches Methanol als einzige Energie- und Kohlenstoffquelle erhalten haben.

8. Pharmazeutische oder dermatologische Zusammensetzung zur topischen Verwendung nach Anspruch 7, wobei die Komagataella durch steigende Methanolkonzentrationen oxidativ gestresst wurde.

9. Pharmazeutische oder dermatologische Zusammensetzung enthaltend Komagataella zur topischen Verwendung nach einem der Ansprüche 6 bis 8 zur Behandlung von Hauterkrankungen, Hautatrophie, Dermatitis, Verbrennungen, Wunden und Verletzungen und zur Wundheilung.

10. Pharmazeutische oder dermatologische Zusammensetzung enthaltend Komagataella zur topischen Verwendung nach Anspruch 9, wobei die Dermatitis ausgewählt ist aus der Gruppe periorale Dermatitis, Dermatitis ulcerosa, atopische Dermatitis, seborrhoische Dermatitis, Neurodermitis, Lichtdermatosen, Xerodermie, Sebostase.

11. Dermatologische Zusammensetzung enthaltend Komagataella zur topischen Verwendung nach einem der vorstehenden Ansprüche, in Form eines Gels, Sprays, Shampoos, einer Salbe, Creme, Lotion (Hautmilch), Paste oder einer Emulsion.

**12.** Dermatologische Zusammensetzung enthaltend Komagataella zur topischen Verwendung nach einem der vorstehenden Ansprüche, wobei die Trockenmasse aus Komagataella lysiert, homogenisiert, zerkleinert oder gepresst wird, oder die Kulturen oder Extrakte oder Biomasse aus Komagataella lysiert, homogenisiert, zerkleinert oder gepresst und anschließend getrocknet werden.

**13.** Dermatologische Zusammensetzung enthaltend Komagataella zur topischen Verwendung nach einem der vorstehenden Ansprüche, wobei Komagataella in Mikrokapseln, Mikropartikel oder Liposomen vorliegen.

**14.** Dermatologische Zusammensetzung enthaltend Komagataella zur topischen Verwendung nach einem der vorstehenden Ansprüche, wobei zusätzlich Antioxidantien, insbesondere Cystein, Ascorbinsäure (Vitamin C), Tocopherole (Vitamin E), Glutathion, Thioredoxin, Liponsäure, Astaxanthin, Canthaxanthin, Lutein, Lycopin, oder Zeaxanthin enthaltend sind.

Figur 1

zu Figur 1 - aus : Kurtzman et. al., Phylogenetic relationships among species of Pichia, Issachenkia and Williopsis determined from multigene sequence analysis, and the proposal of Barnettozyma gen.nov., Lindnera gen.nov. and Wickerhamomyces gen.nov. FEMS Yeast Res 8 (2008) 939-954.

Figur 2

Figur 3a:

Darstellung des Ergebnisses für die Probandin Nr. 13,
Prüfpräparat A (Kontrolle).

Figur 3b:

Darstellung des Ergebnisses für die Probandin Nr. 02,
Prüfpräparat B (Verum).

Figur 4:

Mittelwerte der Intensität der Falten, ermittelt zu Beginn, nach 28 und 56 Tagen der Heimanwendung. Mittelwert ± Standardfehler, Probandin Nr. 02, Prüfpräparat B (Verum).

Figur 5:

Vor Behandlung        nach 2 Wochen        nach 6 Wochen

**Europäisches Patentamt**
**European Patent Office**
**Office européen des brevets**

# EUROPÄISCHER RECHERCHENBERICHT

Nummer der Anmeldung

EP 20 21 5930

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/121522 A1 (GRUBER JAMES VINCENT [US] ET AL) 17. Mai 2012 (2012-05-17) * Ansprüche 1-37; Beispiele 3-4,11 * ----- | 1-14 | INV. A61K8/14 A61K8/9728 A61K36/00 |
| X | WO 2010/011885 A1 (ARCH PERSONAL CARE PRODUCTS L [US]) 28. Januar 2010 (2010-01-28) * Ansprüche 1-20; Beispiele 1-11 * ----- | 1-14 | A61P17/02 A61P17/18 A61Q17/00 A61Q19/08 A61K36/06 |
| X | HEISTINGER LINA ET AL: "Microbe Profile: Komagataella phaffii: a methanol devouring biotech yeast formerly known as Pichia pastoris", MICROBIOLOGY, [Online] Bd. 166, Nr. 7, 1. Juli 2020 (2020-07-01), Seiten 614-616, XP55812527, ISSN: 1350-0872, DOI: 10.1099/mic.0.000958 Gefunden im Internet: URL:https://www.microbiologyresearch.org/docserver/fulltext/micro/166/7/614_micro000958.pdf?expires=1623316707&id=id&accname=sgid024200&checksum=314E74FD18BB5058EE5E598C99F4FAA7> [gefunden am 2021-06-10] * das ganze Dokument * ----- | 1-8 | A61K36/064 C12N1/16 |
| A | EP 3 616 681 A1 (JOHNSON & JOHNSON CONSUMER INC [US]) 4. März 2020 (2020-03-04) * das ganze Dokument * ----- | 1-14 | RECHERCHIERTE SACHGEBIETE (IPC) A61K A61P A61Q C12R C12N |

Der vorliegende Recherchenbericht wurde für alle Patentansprüche erstellt

| Recherchenort | Abschlußdatum der Recherche | Prüfer |
|---|---|---|
| München | 10. Juni 2021 | Nopper, Agathe |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.........................................................................

& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 20 21 5930

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-06-2021

| Im Recherchenbericht angeführtes Patentdokument | Datum der Veröffentlichung | Mitglied(er) der Patentfamilie | Datum der Veröffentlichung |
|---|---|---|---|
| US 2012121522 A1 | 17-05-2012 | AU 2011325934 A1 | 13-06-2013 |
| | | AU 2016203692 A1 | 23-06-2016 |
| | | CA 2817828 A1 | 18-05-2012 |
| | | CN 103635198 A | 12-03-2014 |
| | | CN 103816108 A | 28-05-2014 |
| | | CN 103816188 A | 28-05-2014 |
| | | CN 103820336 A | 28-05-2014 |
| | | CN 103830282 A | 04-06-2014 |
| | | CN 103830383 A | 04-06-2014 |
| | | CN 108619074 A | 09-10-2018 |
| | | EP 2637641 A2 | 18-09-2013 |
| | | EP 2799076 A1 | 05-11-2014 |
| | | EP 2799077 A1 | 05-11-2014 |
| | | EP 2799078 A1 | 05-11-2014 |
| | | EP 2799079 A1 | 05-11-2014 |
| | | EP 2799080 A1 | 05-11-2014 |
| | | EP 3527214 A1 | 21-08-2019 |
| | | JP 6508872 B2 | 08-05-2019 |
| | | JP 2014500872 A | 16-01-2014 |
| | | KR 20130140224 A | 23-12-2013 |
| | | KR 20130141719 A | 26-12-2013 |
| | | KR 20130141720 A | 26-12-2013 |
| | | KR 20140005894 A | 15-01-2014 |
| | | KR 20140018370 A | 12-02-2014 |
| | | KR 20140018371 A | 12-02-2014 |
| | | KR 20180130006 A | 05-12-2018 |
| | | KR 20190086482 A | 22-07-2019 |
| | | US 2012121522 A1 | 17-05-2012 |
| | | US 2014037675 A1 | 06-02-2014 |
| | | US 2014037676 A1 | 06-02-2014 |
| | | US 2014037677 A1 | 06-02-2014 |
| | | US 2014038256 A1 | 06-02-2014 |
| | | US 2014045240 A1 | 13-02-2014 |
| | | US 2019175672 A1 | 13-06-2019 |
| | | US 2020188459 A1 | 18-06-2020 |
| | | WO 2012065121 A2 | 18-05-2012 |
| WO 2010011885 A1 | 28-01-2010 | EP 2299830 A1 | 30-03-2011 |
| | | JP 5687193 B2 | 18-03-2015 |
| | | JP 2011528909 A | 01-12-2011 |
| | | US 2010021532 A1 | 28-01-2010 |
| | | US 2016317433 A1 | 03-11-2016 |
| | | US 2017319469 A1 | 09-11-2017 |
| | | WO 2010011885 A1 | 28-01-2010 |
| EP 3616681 A1 | 04-03-2020 | AU 2019222871 A1 | 19-03-2020 |

EPO FORM P0461

Für nähere Einzelheiten zu diesem Anhang : siehe Amtsblatt des Europäischen Patentamts, Nr.12/82

Seite 1 von 2

**EP 4 014 953 A1**

**ANHANG ZUM EUROPÄISCHEN RECHERCHENBERICHT
ÜBER DIE EUROPÄISCHE PATENTANMELDUNG NR.**

EP 20 21 5930

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-06-2021

| Im Recherchenbericht angeführtes Patentdokument | Datum der Veröffentlichung | Mitglied(er) der Patentfamilie | Datum der Veröffentlichung |
|---|---|---|---|
| | | BR 102019017062 A2 | 10-03-2020 |
| | | CA 3050597 A1 | 29-02-2020 |
| | | CN 110870880 A | 10-03-2020 |
| | | EP 3616681 A1 | 04-03-2020 |
| | | JP 2020033345 A | 05-03-2020 |
| | | KR 20200026113 A | 10-03-2020 |
| | | RU 2019126808 A | 26-02-2021 |
| | | US 2020069563 A1 | 05-03-2020 |

Für nähere Einzelheiten zu diesem Anhang : siehe Amtsblatt des Europäischen Patentamts, Nr.12/82

EPO FORM P0461

Seite 2 von 2

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **AHMED N.** Advanced glycation endproducts - role in pathology of diabetic complications. *Diabetes Res Clin Pract,* 2005, vol. 67, 3-21
- **ZOUBOULIS CC ; MAKRANTONAKI E.** Clinical aspects and molecular diagnostics of skin aging. *Clin Dermatol,* 2011, vol. 29, 3-14
- **KURTZMAN et al.** *FEMS Yeast Res,* 2008, vol. 8, 939-954
- **MEERWALDT R ; LINKS T ; GRAAFF R ; THORPE SR ; BAYNES JW ; HARTOG J et al.** Simple noninvasive measurement of skin autofluorescence. *Ann N Y Acad Sci,* 2005, vol. 1043, 290-8
- **MULDER DJ ; WATER TV ; LUTGERS HL ; GRAAFF R ; GANS RO ; ZIJLSTRA F et al.** Skin autofluorescence, a novel marker for glycemic and oxidative stress-derived advanced glycation endproducts: an overview of current clinical studies, evidence, and limitations. *Diabetes Technol Ther,* 2006
- **BEISSWENGER PJ ; HOWELL S ; MACKENZIE T ; CORSTJENS H ; MUIZZUDDIN N ; MATSUI MS.** Two fluorescent wavelengths, 440(ex)/520(em) nm and 370(ex)/440(em) nm, reflect advanced glycation and oxidation end products in human skin without diabetes. *Diabetes Technol Ther,* 2012, vol. 14, 285-92